# EUROPEAN PATENT APPLICATION

(11) **EP 1 900 754 A1**
(43) Date of publication of application: **19.03.2008**
(21) Application number: 06291457.7
(22) Date of filing: 18.09.2006
(51) Int. Cl.: C07K 19/00, C07K 16/12, C12N 15/62, C07K 16/28

(54) **Fusion proteins comprising two lgG binding domains of streptococcal protein G**

(71) Applicant: INSTITUT NATIONAL DE LA SANTE ET DE LA RECHERCHE MEDICALE (INSERM), 75013 Paris (FR)
(72) Inventor: Van Endert, Peter, 92160 Antony (FR); Kratzer, Roland, 75016 Paris (FR)
(74) Representative: Vialle-Presles, Marie José

(57) **Abstract**

The invention is relative to fusion proteins comprising two IgG binding domains of streptococcal protein G, linked to a polypeptide of interest. These fusion proteins can be complexed with IgG antibodies directed against surface receptors of target cells, allowing their delivery to said target cells.

## Description

The present invention is directed to fusion proteins that can be targeted to various cells, including in particular antigen presenting cells such as dendritic cells.

Vaccines are designed to stimulate adaptive memory immune responses that prevent pathologies, generally by rapid elimination of the pathogen, upon subsequent infection by it. Although most successful vaccines act by stimulating the production of neutralizing antibodies, it is thought that vaccines eliciting efficient adaptive cellular responses are likely to be required to control pathogens such as human immunodeficiency virus (HIV) or hepatitis C virus (HCV) (BERZOFSKY et al., J Clin Invest, 114, 450-62, 2004). Development of efficient strategies for priming and boosting T cell responses is therefore of major interest.

Stimulation of T cell responses requires that relevant antigens gain access to the appropriate intracellular compartments to be broken down to peptides, which can then be loaded on major histocompatibility complex (MHC) class I and/or class II molecules (BRYANT & PLOEGH, Curr Opin Immunol, 16, 96-102, 2004; SHASTRI et al., Annu Rev Immunol, 20, 463-93, 2002). Moreover, peptide presentation by cells providing efficient costimulation is important for priming and boosting T cell responses, a requirement met best by dendritic cells (DCs) (BANCHEREAU & PALUCKA, Nat Rev Immunol, 5, 296-306, 2005). The latter also possess the unique functional capacity of presenting via their MHC class I molecules peptides derived from internalized material, a property referred to as cross-presentation (GUERMONPREZ et al., Annu Rev Immunol, 20, 621-67, 2002). Therefore, exploitation of the unique antigen presentation and T cell priming of DCs is an important objective of current vaccine technologies (ADEMA et al., Curr Opin Immunol, 17, 170-4, 2005; BERZOFSKY et al., J Clin Invest, 114, 450-62, 2004; NESTLE et al., Curr Opin Immunol, 17, 163-9, 2005).

Numerous pathways and strategies that deliver vaccine antigens to professional antigen presenting cells (APCs) including DCs have been developed (FOGED et al., Pharm Res, 19, 229-38, 2002). Attenuated or recombinant microorganisms generally induce efficient T cell stimulation however their use can be limited by vector immunogenicity (DREXLER et al., Curr Opin Biotechnol, 15, 506-12, 2004). Recombinant antigens induce efficient CD4⁺ and antibody responses but require relatively hydrophilic antigens and fail to induce CD8⁺ cytotoxic T cell (CTL) responses (WAGNER et al., Curr Opin Biotechnol, 15, 538-42, 2004). DNA vaccines overcome the latter problem but tend to possess poor efficacy in humans (DONNELLY et al., J Immunol, 175, 633-9, 2005). Finally, synthetic peptide vaccines are easy to produce and administer but result in indiscriminate antigen presentation by professional APCs and non-professional APCs and require knowledge of the human leukocyte antigen (HLA) types of vaccinated individuals (SETTE & FIKES, Curr Opin Immunol, 15,461-70,2003).

In view of these limitations of the most common vaccine strategies, targeting of recombinant antigens to professional APC has recently been considered as an attractive additional strategy of vaccine delivery (BONIFAZ et al., J Exp Med, 199, 815-24, 2004; ERIKSSON et al., Eur J Immunol, 34, 1272-81, 2004; FELNEROVA et al., Curr Opin Biotechnol, 15, 518-29, 2004; PERRIN-COCON et al., J Immunol, 172, 3564-72, 2004; RAMAKRISHNA et al., J Immunol, 172, 2845-52, 2004; SETTE & FIKES, Curr Opin Immunol, 15, 461-70, 2003).

Antigen targeting to professional APCs should have several important advantages, including reduced consumption of recombinant antigen, increased efficiency of T cell stimulation, and diminished undesired tolerizing effects resulting from antigen presentation by non-professional APCs. Some of these expected benefits have indeed been documented in the small number of studies where antigens were targeted to DCs. Very low doses of a monoclonal antibody (mAb) to CD11c, a DC marker in the mouse, were sufficient to induce specific antibody responses (WANG et al., Proc Natl Acad Sci U S A, 97, 847-52, 2000). Ovalbumin and HIV-gag coupled by chemical or genetic strategies to a mAb against DEC-205 or Dectin-1, mouse DC/macrophage marker, elicited very efficient T cell responses (BONIFAZ et al., J Exp Med, 199, 815-24, 2004). Finally, recombinant *B. pertussis* adenylate cyclase fusion proteins that bind to the aMβ2 integrin on DCs induce strong CD4⁺ and CD8⁺ responses (DADAGLIO et al., Int Immunol, 15, 1423-30, 2003; SCHLECHT et al., J Immunol, 173, 6089-97, 2004).

The inventors have tried to use the approach of coupling an antigen of interest with a targeting antibody, as described by BONIFAZ et al (2004, cited above), with various antigens. However, they observed that although this strategy was successful for soluble antigens such as albumin, it did not work in the case of influenza matrix protein I (FluM1), and hypothesized that it was in part due to the hydrophobicity of the antigen. Since many antigens of interest, for instance numerous pathogen structural proteins, are hydrophobic proteins, this represents an obstacle to antigen targeting strategies.

In order to solve this problem, the inventors considered to couple the antigens to antibodies through an immunoglobulin G (IgG) binding domain rather than directly. They choose the IgG binding domain of streptococcal protein G.

Streptococcal protein G is a surface protein from group C or group G streptococci. It has broad IgG binding capacity across various species and isotypes (AKERSTROM et al., J Immunol, 135, 2589-92, 1985). It comprises 2 or 3 (depending on the streptococcal strain) IgG binding domains with high sequence homology (pfam01378 in the CDD database; MARCHLER-BAUER et al., Nucleic Acids Res, 33, D192-6, 2005), known as B1, B2 and B3. Each of these domains folds into a central alpha helix and a four-stranded beta sheet.

Streptococcal protein G has been used in antibody labelling and purification, and its B1 domain has been used as a "solubility-enhancement tag" stabilizing covalently linked proteins for affinity purification and subsequent NMR studies (ZHOU et al., J Biomol NMR, 20, 11-4, 2001).

The inventors constructed recombinant fusion proteins comprising an hydrophobic antigen linked to a single protein G IgG binding domain. They obtained fusion proteins that bound to rabbit IgG. However, these fusion proteins did not form complexes with mouse mAbs of the IgG1 isotype with lower affinity for protein G (AKERSTROM et al., 1985, cited above) and were therefore unlikely to be widely useable. The inventors overcame this problem by the addition of a second protein G IgG binding domain. The fusion proteins thus obtained, comprising a tandem protein G IgG binding domain, were able to form complexes with a variety of antibodies. Using the model antigen ovalbumin, the inventors have also shown that the fusion proteins, when coupled to several antibodies, elicit both CD4⁺ and CD8⁺ T cell responses *in vitro* and *in vivo* at least 100-fold more efficiently than antigen alone, demonstrating the potential of the strategy for vaccination.

The present invention provides a fusion protein comprising:
- an IgG binding moiety consisting of two IgG binding domains of streptococcal protein G placed in tandem arrangement and optionally separated by a peptide linker, and
- a cargo moiety comprising a polypeptide of interest.

A "fusion protein" refers to a protein artificially created from at least two amino-acid sequences of different origins, which are fused either directly (generally by a peptide bond) or via a peptide linker.

Generally, in a fusion protein of the invention, the IgG binding moiety is located N-terminally relative to the cargo moiety. Preferably, said IgG binding moiety and said cargo moiety are fused via a peptide linker.

The two IgG binding domains of streptococcal protein G may be identical or different. Any combination of two domains among the B1, B2, and B3 domains, in any order, can be used. Preferred combinations include B1-B1, B1-B2, B2-B1, and B2-B2.

Preferably, a fusion protein of the invention does not include sequences of streptococcal protein G other than the above-defined IgG binding domains. In particular, it does not include protein G albumin binding domains.

The "cargo moiety" can be any polypeptide that one intends to deliver to a target cell. Said cargo moiety may also be a fusion protein, wherein the polypeptide of interest is associated with additional sequences.

The "polypeptide of interest" may be any polypeptide that is able to induce a biological response of the target cell and/or the organism containing the target cell, as well as any polypeptide that one wishes to test for its ability to induce such a biological response.

Examples of polypeptides of interest are antigenic or potentially antigenic polypeptides. This includes polypeptides against which it is desirable to obtain an antibody response or a T cell response, for instance antigenic polypeptides from pathogenic organisms, such as virus, bacteria, fungi, or protozoa, or from cancer antigens (i. e. antigens specifically associated with cancer cells); this includes also polypeptides containing sequences that one wishes to test for their ability to induce an antibody response or T cell reactivity, for instance candidate self-antigens.

Other examples of polypeptides of interest are polypeptides comprising a sequence of therapeutic interest, for instance a protein drug or pro-drug that one wishes to target to a particular cell type.

It may be advantageous, in particular in cases wherein the polypeptide of interest is an antigenic polypeptide, to fuse an ubiquitin domain to its N-terminal end, in order to enhance its degradation in the proteasome-dependent MHC class I antigen processing pathway (ANDERSSON & BARRY, Mol Ther, 10, 432-46, 2004). If one uses naturally occurring ubiquitin, having a glycine at position 76, and lysines at positions 29 and 48, the presence of an N-terminal ubiquitin molecule may, upon protein translocation into the cytosol, enhance degradation of the polypeptide of interest by cellular proteasome complexes. This enhancing effect on degradation will not occur if one uses an ubiquitin variant wherein both the lysines at positions 29 and 48 are substituted by other amino-acid residues, e.g. arginine or alanine; such an ubiquitin variant may however play a part in the stabilization of the fusion protein. Conversely, non cleavable ubiquitin-antigen junctions, obtained by replacing the C-terminal glycine of the ubiquitin sequence by another amino-acid residue (such as valine or cysteine), can be used if the objective is to induce more potent CTL responses, using the pathway of "ubiquitin fusion degradation".

The cargo moiety may also comprise a labeling polypeptide, allowing to monitor the binding of the fusion protein to the target cell, and its internalization therein; it can also be useful for monitoring of antigen uptake in living cells, following vaccination of laboratory animals. Said labeling polypeptide may be a fluorescent protein, which can be monitored for instance by flow cytometry and fluorescence microscopy. Non limitative examples of fluorescent proteins include: the Green Fluorescent Protein (GFP) and its variants such as the Yellow Fluorescent Protein (YFP); the monomeric red fluorescent protein from *Discosoma* (DsRed). Epitope tags, (for instance the HA tag or the FLAG tag) which are generally smaller than fluorescent tags can also be used as labeling polypeptides.

The labelling polypeptide can be located anywhere within the cargo moiety, i.e N-terminally or C-terminally relative to the polypeptide of interest. However, in the case wherein enhanced degradation of the polypeptide of interest by ubiquitin fusion is desired, the ubiquitin domain should be fused directly to the N-terminal end of the polypeptide of interest. In this case, the label should be preferably placed N-terminal of the ubiquitin domain.

Peptide linkers may be employed to separate two or more of the different components of a fusion protein of the invention. In particular, peptide linkers will advantageously be inserted between the two IgG binding domains in the IgG binding moiety, and between the IgG binding moiety and the cargo moiety. Peptide linkers are classically used in fusion proteins in order to ensure their correct folding into secondary and tertiary structures. They are generally from 2 to about 50 amino acids in length, and can have any sequence, provided that it does not form a secondary structure that would interfere with domain folding of the fusion protein.

Optionally, the fusion proteins of the invention may also comprise other components, such as short sequences allowing their targeting to specific sub-cellular compartments.

The size of the cargo moiety may vary from a few kDa to a few hundred kDa. Preferably, it will vary from 10 kDa to 150 kDa.

A fusion protein of the invention can be produced by expressing a recombinant DNA molecule encoding said protein in an appropriate host-cell.

The invention also provides a recombinant polynucleotide, in particular a recombinant DNA, comprising a sequence encoding a fusion protein of the invention. If necessary, a recombinant polynucleotide of the invention may further comprise a sequence encoding a signal peptide, allowing the secretion of the fusion protein by the host-cell. The choice of an appropriate signal peptide depends, in particular, on the host-cell that one intends to use.

The invention also encompasses recombinant vectors, in particular expression vectors, comprising a polynucleotide of the invention, and host cells containing said recombinant vectors.

The invention also provides a method for producing a fusion protein of the invention, wherein said method comprises a) culturing an host-cell comprising a recombinant polynucleotide of the invention and b) recovering the fusion protein of the invention produced by said host-cell.

A broad variety of expression systems for producing recombinant proteins is available in the art, and can be used to produce the fusion proteins of the invention. This includes prokaryotic expression systems, as well as eukaryotic expression systems, such as yeast expression systems, plant cells expression systems, insect cells expression systems or mammalian cells expression systems.

Due to their IgG binding properties he fusion proteins of the invention can easily be purified by a single chromatographic step, on an IgG column.

The present invention also provides a complex of a fusion protein of the invention with a mammalian IgG antibody.

This complex is formed by non-covalent binding between the two IgG binding domains in the fusion protein and the IgG antibody.

Said IgG antibody is preferably a monoclonal antibody directed against a surface receptor of the target cell (for this reason said antibody will be also designated hereinafter as "targeting antibody"). Its choice thus depends on the selected target cell.

By way of non-limitative examples, if the target cells are peripheral blood monocytes, one can use antibodies directed against LFA-1 (CD11a/18), CR3 (CD11b/CD18), CD14, Fc receptors, mannose receptor; if the target cells are B lymphocytes one can use for instance antibodies directed against CD19, or CD21; if the target cells are T lymphocytes one can use for instance antibodies directed against CD3, CD4, or CD8.

In the case wherein the polypeptide of interest is an antigenic polypeptide that one wishes to target to dendritic cells, one can use for instance antibodies directed against the surface receptors DEC-205, DC-SIGN or Dectin-1.

In particular, one can use antibodies directed against the receptor DC-SIGN. This receptor has many features that render it an attractive target for antigen delivery: almost exclusive expression on DCs, high expression on immature DCs with high capacity of antigen internalization and processing, and efficient internalization due to several internalization motifs (ENGERING et al., J Immunol, 168, 2118-26, 2002). Preferred anti-DC-SIGN antibodies are those directed against against the C-terminal ligand binding domain of DC-SIGN. A particularly preferred antibody is the monoclonal antibody RK113D2. An hybridoma cell line producing said antibody has been deposited under Budapest Treaty in the CNCM (Collection Nationale de Cultures de Microorganismes, 25 rue du Docteur Roux 75724 Paris Cedex 15 France), on August 2, 2006 under No. CNCM 1-3661.

The present invention also comprises compositions comprising a fusion protein, a monoclonal antibody, or a complex of the invention.

According to a preferred embodiment, a composition of the invention may comprise two or more different complexes of the invention.

For instance, it may comprise two or more different complexes of a same fusion protein of the invention with two or more different antibodies. Said different antibodies may be directed against different surface receptors of the target cell, or against the same receptor. This may be helpful, in the case of compositions used for vaccination, to reduce production against idiotypic (or species-specific) determinants of targeting antibodies. It may also comprise two or more different complexes of different fusion proteins of the invention with a same targeting antibody. This may allow to optimize vaccine responses, by inducing T cell responses with broad specificity, directed against multiple antigens, which will be helpful to protect from diseases such as infection by HIV or HCV.

According to a preferred embodiment, the compositions of the invention are pharmaceutical compositions, for instance vaccines for treating or preventing an infectious disease, in particular a viral disease, or vaccines for treating cancer.

These pharmaceutical compositions generally comprise, besides the active principle consisting of the fusion protein, the monoclonal antibody, or the complex of the invention, pharmaceutically acceptable carriers and additives.

By way of example, for preparing a pharmaceutical composition comprising a complex of the invention, of the invention the fusion protein and the targeting antibody are mixed in a pharmaceutically acceptable medium (for instance physiological saline) which may optionally comprise suitable additives (for instance stabilizing agents such as glycerol). The molar ratio: fusion protein/targeting antibody can vary. However, a molar excess of targeting antibody is preferable, in particular in the case of compositions to be administered *in vivo.* A preferred ratio: fusion protein/targeting antibody is of between 1/2 to 1/5, advantageously of about 1/3.

The compositions of the invention can be administered by any route that results in contact between the fusion protein/targeting antibody complex and the target cell. Although intravenous administration may be suitable, preferred routes of administration are intradermic injection, and still more preferably sub-cutaneous injection, for targeting cutaneous dendritic cells and/or Langerhans cells, or for antigen delivery to regional lymph nodes. Intratumoral or peritumoral injection can also be used in the case of cancer vaccines.

Another object of the invention is a method for delivering a fusion protein of the invention to a target cell *in vitro,* wherein said method comprises forming a complex between a fusion protein of the invention and a mammalian IgG antibody directed against a surface receptor of said target cell, and contacting said complex with said target cell.

The method of the invention can be used in particular for preparing dendritic cells vaccines. In this case, a fusion protein of the invention comprising an antigen to be administered to a patient is complexed with a targeting antibody directed against a dendritic cell surface receptor. The complex fusion protein/targeting antibody is incubated *in vitro* with dendritic cells previously obtained from said patient, and the DCs having internalized the complex are recovered. They can then be returned to the patient.

The method of the invention can also be used for *in vitro* tests of CD8+ T cell reactivity against candidate antigens. In this case, a fusion protein of the invention comprising the candidate antigen to test is complexed with a targeting antibody directed against an antigen presenting cell present among lymphocytes in a sample of peripheral blood previously obtained from the individual to be tested. This antigen presenting cell will be in most cases a monocyte, but may also be one of the rare circulating dendritic cells.

The complex fusion protein/targeting antibody is contacted with a sample of peripheral blood cells previously obtained from a subject to be tested and the recognition of the antigen delivered as a fusion protein is analyzed by standard methods that measure cytokine production (Elispot or ELISA assays), proliferation (³H thymidine uptake, CFSE dilution), or cytotoxicity (⁵¹Cr release).

The present invention will be further illustrated by the following additional description, which refers to non-limitative examples illustrating the properties of fusion proteins of the invention.

### EXAMPLE 1 : CONSTRUCTION OF FUSION PROTEINS CONTAINING TANDEM IgG BINDING DOMAINS OF STREPTOCOCCAL PROTEIN G

Our aim was to produce antigens derived from viral pathogens in a form allowing for versatile targeting to surface receptors on professional APCs, and inducing efficient priming of T cell responses.

Four proteins derived from viruses causing considerable human pathology were chosen : influenza matrix 1 (flu M1) (NAYAK et al., Virus Res, 106, 147-65, 2004), hepatitis C virus (HCV) core protein (RAY & RAY, FEMS Microbiol Lett, 202, 149-56, 2001), human immunodeficiency virus (HIV)-1 nef (JOSEPH et al., Curr HIV Res, 3, 87-94, 2005), and human T lymphotropic virus (HTLV)-1 tax (GATZA et al., Oncogene, 22, 5141-9, 2003). In addition, we expressed the frequently used model antigen ovalbumin (OVA) in order to take advantage of available transgenic mouse strains expressing OVA-specific T cell receptors (BARNDEN et al., Immunol Cell Biol, 76, 34-40, 1998; HOGQUIST et al., Cell, 76, 17-27, 1994), which facilitate studies of antigen presentation *in vitro* and *in vivo* (see below). With the exception of OVA, all of these proteins are hydrophobic. FluM1 and truncated HCV core, when expressed in insect cells, can only be recovered upon solubilization in strong detergents or denaturing agents such as 8 M urea. Due to the extreme hydrophobicity of its C-terminus, HCV core was expressed truncated by 15 C-terminal residues.

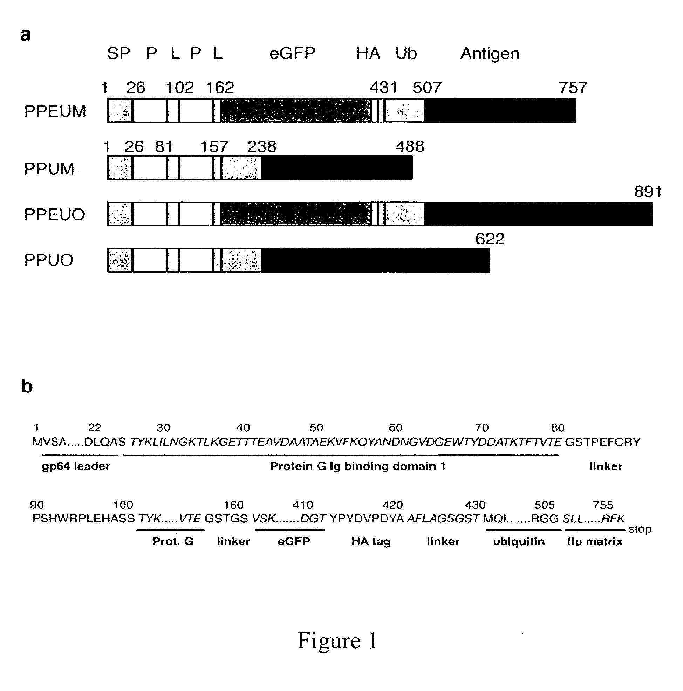

The other components of the fusion proteins are displayed in Fig. 1a and Fig. 1b. The leader peptide derived from an insect cell protein had previously been shown to result in efficient secretion of recombinant proteins by insect cells (FOURNEAU et al., J Immunol Methods, 285, 253-64, 2004). Tandem Ig binding domains of protein G, which has broad Ig binding capacity across various species and isotypes (AKERSTROM et al., J Immunol, 135, 2589-92, 1985), were inserted to allow for binding of the fusion proteins to antibodies. Ubiquitin (Ub) was inserted upstream of the chosen antigens since it can enhance proteasomal degradation of proteins linked to its carboxyterminus (QIAN et al., J Biol Chem, 277, 38818-26, 2002). Fusion proteins were produced both without and with an enhanced green fluorescent protein (eGFP) domain introduced between the second protein G domain and Ub, so as to facilitate monitoring of fusion protein binding to, and internalization by, cells using flow cytometry and microscopy. Linkers of various lengths separate the different components.

Hereinafter, the different fusion proteins will be designated according to the following nomenclature: letters represent the following elements: P, protein G IgG binding domain 1; E, eGFP; U, ubiquitin; M, FluM1; O, ovalbumin; N, HIV-nef. By way of example, PPEUM designates a fusion protein comprising the duplicated protein G IgG binding domain, an eGFP domain, an ubiquitin domain, and the FluM1 antigen. In some of the proteins, the Arg residue in position 48 of the ubiquitin domain has been substituted by a Lys residue; these proteins will be identified by K-48.

Figure I a shows a schematic representation of the precursors (i.e including the signal peptide) of different fusion proteins. Boxes drawn to scale represent the components of selected proteins. The main components are designated by letters, according to the nomenclature indicated above. Other components are designated as follows: SP, signal peptide; L, linker; P, protein G Ig binding domain 1; HA, hemagglutinin tag.

Fig. 1b shows as an example a more detailed view of a complete fusion protein, comprising the precursor of the FluM1 protein with the eGFP label (PPEUM). The different elements are indicated below the amino acid sequence, residues are numbered above it. gp64 leader, signal peptide of the gp64 glycoprotein (followed by an Ala-Ser dipeptide corresponding to a NheI site); eGFP, enhanced green fluorescent protein; HA tag, hemagglutinin tag; flu matrix, influenza matrix protein 1.

### Construction of plasmids encoding the fusion proteins.

Complementary DNA constructs encoding the various fusion proteins were assembled stepwise from polymerase chain reaction (PCR) products, generated using high fidelity enzymes (Advantage HF, Clontech, Mountain View, CA) and cloned in the vector pCR Blunt (Invitrogen, Cergy Pontoise, France).

First, a cassette encoding enhanced green fluorescent protein (eGFP) linked to human ubiquitin (Ub) was amplified by PCR from vector pGene eGFP/Ub, (VALMORI et al., J Exp Med, 189, 895-906, 1999), using primers including ScaI (5') and SacII (3') restriction sites. In parallel, an Ub cDNA was amplified from that plasmid, using primers with the same restriction sites. Both products were cloned in pCR Blunt.

In the second step, cDNAs encoding the five chosen antigens were amplified from different plasmids with primers including a SacII site in the 5' primer, and were also cloned into pCR-Blunt.

In the third step, a cDNA encoding the first immunoglobulin-binding domain of protein G was constructed by inserting annealed complementary oligonucleotides including NheI (5') and ScaI (3') sites into the appropriately digested vector pCR blunt.

In the fourth step, a sequence encoding the gp64 baculovirus protein signal peptide was excised from the published vector pAcUW51DRB1*0404 (FOURNEAU et al., J Immunol Methods, 285, 253-64, 2004) using BgIII (5') and EcoRI (3'), and ligated into the baculovirus transfer vector pVL1392. All plasmids were sequenced to ensure absence of errors introduced in the PCR.

Fusion proteins were then assembled in the following manner. First, the antigen encoding cDNAs were excised from pCR Blunt using SacII (5') and an XbaI site in the multiple cloning site of the vector, and ligated into the appropriately digested pCR Blunt plasmids containing the eGFP-Ub cassette, or Ub alone. The resulting cassettes, encoding the antigens preceded by eGFP-Ub or Ub alone, were then transferred as ScaI (5')/XbaI(3') fragments into pCR Blunt containing the protein G domain sequence. Next, a second protein G domain encoding sequence was excised from pCR Blunt using NheI (5') and an Xba I site in the pCR Blunt multiple cloning site, and ligated to the various plasmids containing all downstream elements, opened by Nhel only (NheI and XbaI generate compatible ends). In the final step, the entire cassette including two protein G domains was transferred as NheI/XbaI fragment into the pVL1392 plasmid containing the gp64 signal peptide sequence so that the fusion proteins were joined in frame to the signal peptide. To produce fusion proteins with Lys 48 in Ub, we used the Quikchange mutagenesis kit (Stratagene, Amsterdam, The Netherlands), followed by sequencing.

After assembly, the plasmids encoding the fusion proteins were transfected into Sf9 insect cells to produce recombinant baculoviruses.

### EXAMPLE 2 : EXPRESSION AND PURIFICATION OF THE FUSION PROTEINS.

Recombinant baculoviruses were produced by Cellfectin^{™} mediated co-transfection of the pVL1392-based plasmids (5 µg), described above, with 200 ng Baculogold^{™} viral DNA (reagents from Invitrogen, Cergy Pontoise, France) into 9 x 10⁵ adherent Sf9 *Spodoptera frugiperda* insect cells. Recombinant baculoviruses were identified by PCR analysis of infectious supernatants derived from standard plaque assays.

For production of recombinant proteins, 4 x 10⁷ Sf9 or Hi5 (*Trichoplasia Ni*) insect cells were seeded in 175 cm² tissue culture flasks (Nalgene-Nunc, Roskilde, Denmark). Forty-eight h later, the cells were infected for I h at a multiplicity of infection of 2 with infectious supernatant, which was then replaced by serum-free Ex-Cell 420 (JRH Biosciences, Andover, U.K.; for Sf9) or Ex-Cell 405 (JRH; for Hi5) medium (20 ml per flask).

To find out whether the fusion proteins were secreted by baculovirus-infected Sf9 cells, we examined cell culture supernatants, removed at various time points after infection, by immunoblot : standard denaturing SDS-PAGE was performed using mini-gel equipment (Mini-Protean 3, BioRad, Hercules, CA) and Tris-glycin buffers. For direct visualization of separated proteins, gels were stained using Simply Blue^{™} stain (Invitrogen). For immunoblot analysis, proteins were transferred for 1h at 80V onto nitrocellulose using Mini Transblot cells (BioRad) and a buffer containing 10 mM CAPS and 10% methanol. Membranes were blocked overnight with 2% non fat dry milk in TBS buffer (Tris 20 mM, NaCl 150 mM, pH 7.5). Primary and secondary antibodies were diluted in TBS with 0.05% Tween 20 (Sigma, Saint-Quentin Fallavier, France), and antibody binding was visualized by enhanced chemoluminescence (ECL Plus, Amersham Biosciences), which was read using a Fujifilm LAS 1000+ system (Fujifilm, Saint Quentin en Yvelines, France). The following primary antibodies were used: supernatant (1:50) of hybridoma M2-1C6-4R3 (ATCC, Manassas, VA) specific for influenza matrix protein 1; rabbit antibodies recognizing peptide 33-43 of HCV core (1:1000; QED Biosciences, San Diego, CA); supernatant (1:50) of hybridoma 43/106/63/19 recognizing HIV nef; supernatant (1:10) of hybridoma 168A51-42 specific for HTLV1 tax). Horse radish peroxidase-coupled goat anti-mouse antibodies or anti-rabbit antibodies (Jackson Research, Cambridgeshire, U.K.), respectively, were used as secondary reagents.

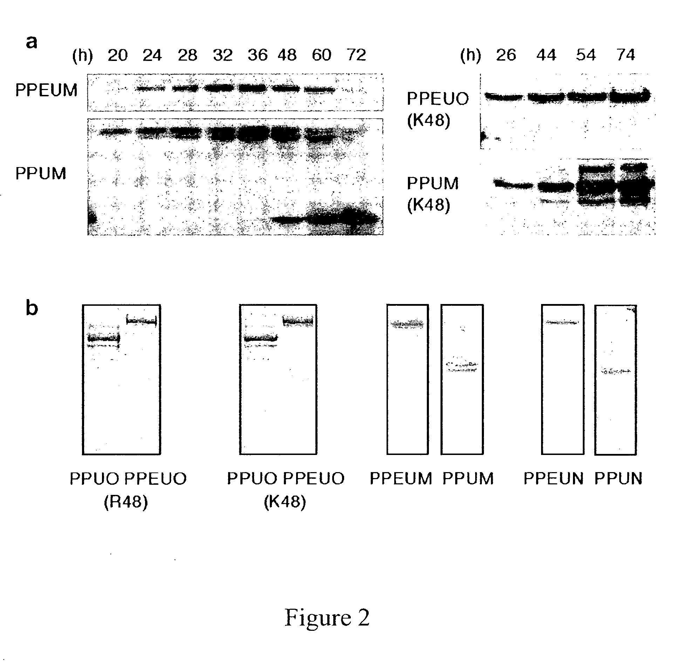

Figure 2a shows an immunoblot analysis of insect cell culture supernatants removed after the number of hours shown above the panels.

For all the constructs, secreted protein was detectable in serum-free culture supernatants, although the time point when maximum concentrations were reached varied between the proteins. Immunoblot analysis revealed that the secreted proteins were subjected to degradation after longer culture periods. However, it was possible to establish, for all proteins, time points at which supernatants containing intact protein but no or little degraded material were harvested.

To obtain purified fusion proteins, larger volumes of supernatants were passed over a rabbit Ig immunoaffinity column produced by coupling Protein G-purified immunoglobulins (Ig) from a non immunized rabbit to cyanobromideactivated Sepharose 4B (Amersham Biosciences, Buckinghamshire, U.K.).

In a typical large scale purification of a fusion protein, 100 to 200 ml of Sf9 cell supernatant was passed at 4°C over a 3 ml rabbit-Ig/Sepharose 4B column. After washing with PBS with 10% glycerol, bound protein was eluted using a buffer containing 20 mM N-cyclohexyl-3-aminopropanesulfonic acid (CAPS) (pH 11.5) and 1% 3-[(3cholamidopropyl) dimethylammonio]-1-propane sulfonate (CHAPS). Eluted fractions were immediately neutralized with 100 mM Tris pH 6.8, and fractions containing the fusion proteins were identified by SDS-PAGE followed by Coomassie blue staining.

Fractions of interest were pooled, dialyzed against PBS with 10% glycerol, and stored in aliquots at -80°C.

Figure 2b shows the SDS-PAGE analysis of the indicated proteins purified by immunoaffinity chromatography.

The yield for different fusion proteins is given in Table I below. Fusion protein nomenclature is as indicated in Example 1 above. The molecular weight and pl are those of the expressed antigen. "E" indicated between brackets means that the protein was constructed with and without the eGFP domain; the numbers between brackets correspond to the yields for proteins without the eGFP domain.

**Table 1**

| Code | Antigen | Molecular Weight | pl | Yield (g/ml) |
|---|---|---|---|---|
| PP(E)UM | Flu M1 | 27.7 | 9.6 | 1.1-2.2 (1.7) |
| PP(E)UN | HIV nef | 23.2 | 5.8 | 1.1 (1.4) |
| PP(E)UC | HCV core | 19.1 | 12.2 | 1.1(N.D.) |
| PPEUT | HTLV-1 tax | 39.3 | 6.5 | 5.6 |
| PP(E)UO | OVA | 42.8 | 5.0 | 1.1 (2.4) |
| PP(E)UO-K48 | OVA | 42.8 | 5.0 | 2.9(1.1) |

SDS-PAGE analysis of eluates demonstrated that >90% pure full-length fusion proteins could be recovered from serum-free culture supernatants by this single step immunoaffinity chromatography procedure (Fig. 2b). Yields were between 1 and 3 µg per ml supernatant for most proteins, but higher for the tax fusion protein (Table 1). Stability tests revealed that purified fusion proteins (specifically PPEUM) showed some precipitation in low salt buffers but were stable for prolonged periods at room temperature when suspended in PBS containing 10% glycerol (not shown), which was therefore chosen for further experimentation.

### EXAMPLE 3: PRODUCTION OF MONOCLONAL ANTIBODIES TO HUMAN DC-SIGN

To test the feasibility of targeting the fusion proteins to APCs, we selected the C-type lectin DC-SIGN, a receptor with many features that render it an attractive target for antigen delivery: almost exclusive expression on DCs, high expression on immature DCs with high capacity of antigen internalization and processing, and efficient internalization due to several internalization motifs (ENGERING et al., J Immunol, 168, 2118-26, 2002). For use as immunogen to produce monoclonal antibodies with specificity for human DC-SIGN, we generated an Ig/DC-SIGN fusion protein that consisted of a signal peptide preceded mouse IgG2b Fc domain linked to the extracellular domain of DC-SIGN. This order of assembly was chosen in order to maximize the odds of obtaining antibodies against the C-terminal ligand binding domain of DC-SIGN, a type II transmembrane protein (GEIJTENBEEK et al., J Leukoc Biol, 71, 921-31, 2002).

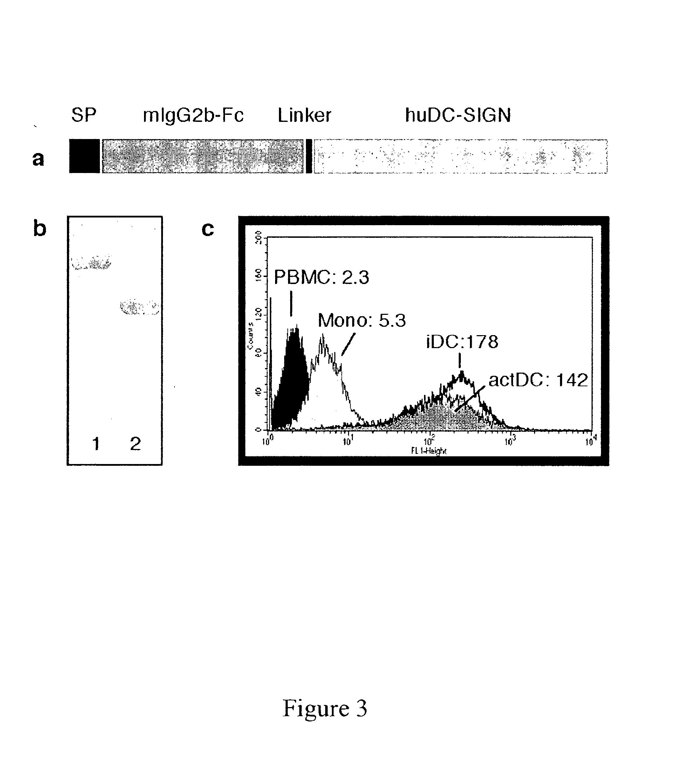

This Ig/DC-SIGN fusion protein is schematized on Figure 3a.

### Production of the recombinant Ig/DC-SIGN

The sequence encoding the extracellular part of human DC-SIGN (residues 61 - 404) was PCR amplified from cDNA of human DCs (a gift of F. Geissman, Paris, France) with primers including BamHl (5') and Sall (3') restriction sites, and inserted into the Drosophila expression vector pRmHA3 (BUNCH et al., Nucleic Acids Res, 16, 1043-61, 1988) containing a metallothionein promoter, previously modified by insertion of a sequence encoding the gp64 signal peptide followed by BamHI and NheI sites. In parallel, a fragment encoding constant domains 2 and 3 of mouse IgG2b was amplified from cDNA of C57/BL6 splenocytes, using primers containing NheI (5') and BamHI (3') sites, and inserted into the pRmHa3 plasmid downstream of the sequences encoding the signal peptide, and upstream of the DC-SIGN fragment. The resulting plasmid (2 µg) was transfected together with a plasmid conferring neomycin resistance (66 ng) into S2 *Drosophila* cells cultured in Ex-Cell 420 medium with 2% FCS, using Cellfectin^{™} liposomes. Cells were selected with 1.5 mg/ml G418 (PAA, Les Mureaux, France)) for 3 wks and subsequently maintained in 0.3 mg/ml G418. To obtain large quantities of the fusion protein, cells were transferred into 350 ml Celline^{™} two compartment culture flasks (Integra Biosciences, Chur, Switzerland)), and cultured at high concentrations in Ex-Cell 420 medium supplemented with 1 mM CuSO₄ for periods of up to four weeks, following the instructions of the manufacturer. Supernatants were recovered every third day and stored at -20°c until purification. To purify recombinant Ig/DC-SIGN, pooled supernatants were passed over a 5 ml protein G column (HiTrap Protein G, Amersham Biosciences). Eluted material was neutralized, dialyzed against PBS and stored in aliquots at -80C until use. The described protocol yielded about 30 µg of >90% pure protein per ml of supernatant.

Figure 3b shows the purified Ig/DC-SIGN fusion protein (about 4 µg), analyzed by SDS-PAGE and Coomassie blue staining (lane 1). The control in lane 2 is an analogous fusion protein, in which human DC-SIGN is replaced by its smaller mouse counterpart.

### Production of monoclonal antibodies

The purified Ig/DC-SIGN fusion protein was injected in Balb/c mice for production of monoclonal B cell hybridomas.

Female 6 wk old Balb/c mice were immunized 3 times with 100 µg fusion protein, first emulsified in complete Freund's adjuvant and injected s.c., then emulsified in incomplete Freund's adjuvant s.c., and finally in PBS and injected i.p. Three days after the final injection, hyperimmunized spleen cells were fused to P3X63XAg8.653 myeloma cells (obtained from ATCC), and resulting hybridomas were selected by aminopterin following standard protocols. Hybridoma supernatants were screened for secretion of desired antibodies starting day 10 after the fusion, and positive hybridomas were cloned immediately by limiting dilution. Two assays were used for screening, an enzyme-linked immunosorbent assay (ELISA) and a cell surface-staining assay. In the initial ELISA test, supernatants were screened for binding to plastic adsorbed Ig/DC-SIGN immunogen relative to an IgG2b isotype antibody control. Clones binding exclusively to the fusion protein were further tested in a cell bound immunosorbent assay adopted from Dörken et al. (DORKEN et al., J Immunol Methods, 88, 129-36, 1986). Fourteen hybridomas showing bright staining of immature human DCs but not of control lymphoblastoid B cells were cloned and injected into Balb/c mice for ascites production. Monoclonal antibodies (mAb) were purified by immunoaffinity chromatography on a protein G column, and dialyzed against PBS before use.

Twelve mAb recognizing recombinant Ig/DC-SIGN coated on plastic plates and human DCs but not an isotype mAb control and control human B cells were selected for further studies.

Figure 3c shows a representative staining with one of these mAb (RK209, IgG1). Human PBMC, monocytes, immature (iDC) or LPS-activated (actDC) DC were stained with mAb RK209 followed by FITC-labeled goat antibodies to mouse Ig, and analyzed in a flow cytometer. The numbers are mean fluorescence intensities (MFI).

In accordance with published data for other mAb recognizing DC-SIGN (GEIJTENBEEK et al., Cell, 100, 575-85, 2000), the mAb shows strong staining of immature DCs and somewhat lower staining of LPS-activated DCs, while human monocytes display negligible staining, and peripheral blood mononuclear cells are not stained at all. Addition of recombinant Ig/DC-SIGN but not of an isotype mAb control inhibited staining of DCs (not shown), demonstrating that the mAb bound to cell surface DC-SIGN rather than to Fc receptors.

### Selection of a monoclonal antibody internalized by DCs

For the proteins to possess potential as vaccine antigens, binding to the DC cell surface must be followed by internalization into antigen processing compartments (SAVEANU & VAN ENDERT, Nat Immunol, 6, 7-8, 2005). Although DC-SIGN has several internalization motifs, internalization has been shown to depend on the DC-SIGN domain recognized by the used antibody (ENGERING et al., J Immunol, 168, 2118-26,2002).

Therefore we tested internalization of the panel of anti-DC-SIGN mAb described above.

Human DCs were prepared according to SALLUSTO et al. (SALLUSTO & LANZAVECCHIA, J Exp Med, 179, 1109-18, 1994). Monocytes were purified from fresh human blood by positive (CD14) selection, using paramagnetic microbeads (Miltenyi, Paris, France). Monocytes were cultured in RPMI supplemented with 10% male human AB serum, 250 ng/ml GM-CSF (Leucomax, Schering-Plough, Levallois-Perret, France) and 10 ng/ml IL-4 (R&D Systems, Abingdon, U.K.), with replacement of 50% of the medium every other day. According to flow cytometric analysis with several DC and activation markers, cells had acquired a DC phenotype and were used for experimentation from day 6. Staining for flow cytometric analysis was generally carried out in 96 well round bottom plates at 4°C in PBS with 1.5% BSA and 0.05% NaN3; cells were washed twice with 0.2 ml buffer between different incubations. All flow cytometric analyses were performed using a FACSCalibur (Becton Dickinson) machine.

To assess internalization of anti DC-SIGN mAbs, DCs were first incubated for 1h at 4°C with 10 µg/ml of mAbs in FACS buffer without NaN₃. After two washings, cells were stained for 30 min at 4°C with 10 µg/ml fluoresceine isothiocyanate (FITC) labeled goat antibodies to mouse Ig (Southern Biotech, Birmingham, Al), and then incubated for 0, 20 or 60 min at 37°C. Cells were then washed, fixed in 1% paraformaldehyde (Sigma) for 20 min at room temperature and finally stained with 5 µg/ml phycoerythrin (PE) labeled goat antibodies to mouse Ig (Beckman Coulter, Roissy, France).

In flow cytometric analysis, the green fluorescent signal reflected the sum of cell surface and internalized anti-DC-SIGN mAb, while the red fluorescent signal reflected mAb remaining on the cell.

Among the panel of anti-DC-SIGN mAb indicated above, three including clone RK113 were internalized after binding to the DC surface at 4°C.

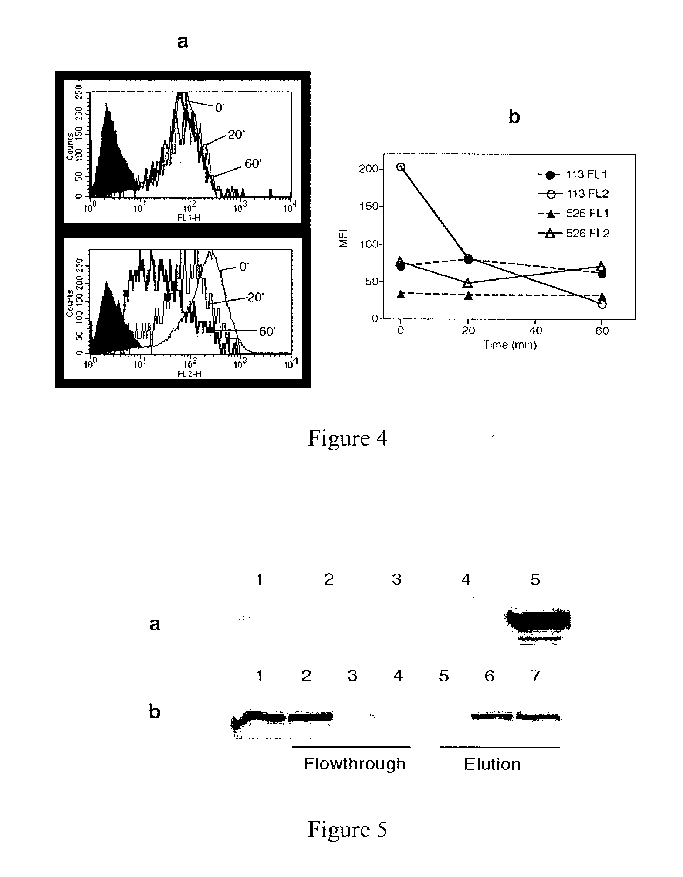

Figure 4a shows the results for RK113, representative of this group of three mAbs. The green fluorescent signal (FL1, top panel) corresponds to total cell-associated mAb RK113, while the red signal (FL2, bottom panel) corresponds to cell surface associated mAb RK113. The amount of mAb detectable on the cell surface decreased rapidly at 37°C, while the total amount of cell-associated mAb changed little over the 60 min observation period. Therefore, while RK113 mAb was rapidly internalized, little or no intracellular degradation appeared to occur in this period.

Figure 4b shows a quantitative evaluation of an equivalent experiment in which, in addition to the internalized anti-DC-SIGN mAb 113, the anti-DC-SIGN mAb RK526, representative the group of non-internalized mAbs, was used. 90% of RK113, was internalized within 1h, while the cell surface-associated amount of RK526 changed little in the same period.

### EXAMPLE 4: BINDING OF THE FUSION PROTEINS TO POLYCLONAL AND MONOCLONAL ANTIBODIES

To be useful for mAb-mediated targeting to various cell surface receptors, the fusion proteins had to possess sufficient affinity for binding to IgG of different isotypes and derived from various species.

Figure 5a shows the results of analysis by immunoblot with a mAb specific for FluM1 during purification of PPEUM from 300 ml of culture supernatant using a rabbit Ig/Sepharose column as described in Example 2 above.

Ten µl (0.003% of the total) of starting supernatant (lane 1), flow through (lane 2), or wash steps (lanes 3 and 4), and 3.3 µl of the elution volume (0.06% of the total) were analyzed. The strong enrichment shows that the fusion protein binds to rabbit IgG with high affinity.

To further examine the binding to IgG of the fusion proteins comprising the tandem protein G IgG-binding domain, 2.6 µg of protein PPEUM in 100 µl PBS/glycerol were incubated for 3 h at 4°C on a rotating wheel with 10 µl of Sepharose beads coupled to the anti-DC-SIGN mAb RK43 or to rabbit Ig (both coupled at 5 mg/ml). The beads were centrifuged for 2 min at 700 x *g*, washed once with 0.5 ml PBS, and eluted three times with 50µl 20 mM CAPS, 1% CHAPS (pH 11.5). Ten µl aliquots of the supernatant, pooled elutions, and resuspended beads were then analyzed by SDS-PAGE followed by immunoblotting with a mAb specific for FluM1.

The results are illustrated by Figure 5b, for fractions of bead supernatant (lanes 2 to 4) and elutions (lanes 5 to 7) incubated with beads coated with glycin (lanes 2 and 5), anti-DC-SIGN mAb RK43 (isotype IgG1, lanes 3 and 6), or polyclonal rabbit IgGs (lanes 4 and 7). Lane 1 shows a 10 µl aliquot of PPEUM before incubation with beads.

These results show that incubation of the PPEUM protein with rabbit IgG-coated beads resulted in quantitative removal of the fusion protein from the supernatant, while incubation with an immobilized mouse IgG1 mAb resulted in near complete removal.

Equivalent results were obtained with other murine mAb of the IgG1, IgG2a and IgG2b isotypes, and with human, goat and rat polyclonal Ig (not shown). Therefore, the tandem protein G IgG-binding domain was sufficient to confer high affinity for Ig of several isotypes and from several species.

### EXAMPLE 5: BINDING OF mAb/FUSION PROTEIN COMPLEXES TO HUMAN DCS, AND INTERNALIZATION OF mAb/FUSION PROTEIN COMPLEXES.

### Binding, of fusion protein/mAb complexes

To examine binding of the fusion proteins to the cell surface, 1x10⁵ purified DCs, pre-incubated or not for 60 min at 4°C with 10 µg/ml anti-DC-SIGN mAb RK113, or with 10 µg/ml control mAb 148.3 specific for intracellular TAP1 peptide transporters (MEYER et al., FEBS Lett, 351, 443-7, 1994), were incubated for 60 min with 5 µg/ml of the fusion protein at 4°C in complete FACS buffer. Cell associated eGFP was then measured by FACS.

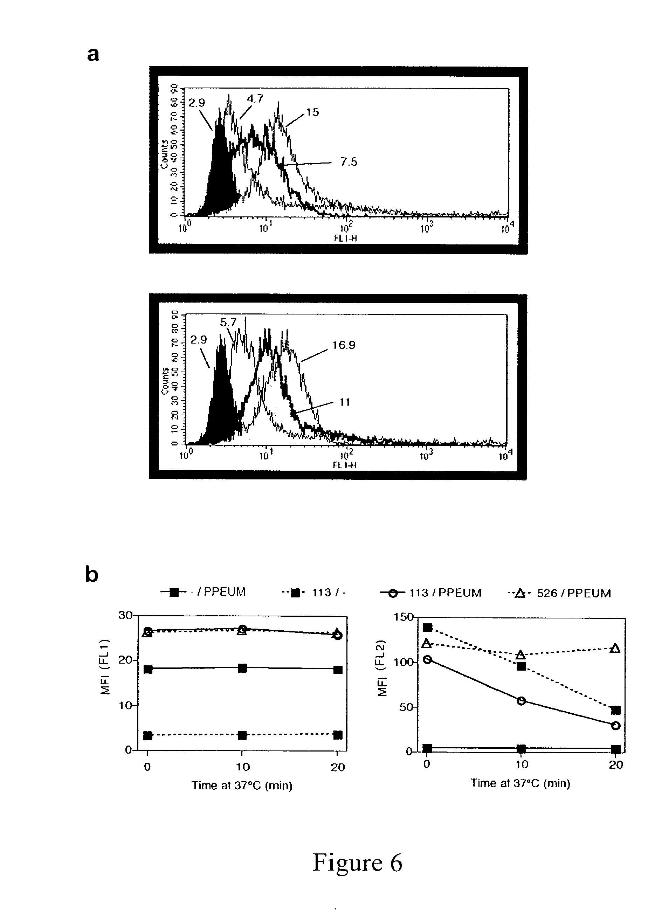

The results are shown in Figure 6a. In the experiment illustrated by the top panel, DCs were incubated with 5 µg/ml PPEUM alone (thick line), or with preformed complexes between PPEUM (5 µg/ml) and a control mAb (10 µg/ml, thin line) or RK113 (10 µg/ml, grey filled profile). Background is shown as black filled profile. Numbers correspond to mean fluorescence intensity (MFI). The bottom panel shows a similar experiment, however in this case cells were pre-incubated for 1h with mAb RK113 (10 µg/ml) before exposure to PPEUM alone (grey filled profile), or to complexes between PPEUM and the control mAb (thin line) or RK113 (thick line).

Incubation of DCs with PPEUM alone resulted in significant staining. PPEUM binding was doubled when DCs were incubated with PPEUM/RK113 complexes, and reduced by more than 50 percent upon incubation with control mAb/PPEUM complexes. The latter observation suggested that free protein G domains were involved in binding of free PPEUM to DCs; most likely the fusion protein binds to human Ig adsorbed to the DC surface. Conversely, increased binding of PPEUM/RK113 complexes could only be due to recognition of DC-SIGN receptors by the mAb.

When DCs pre-coated with mAb RK113 were incubated with PPEUM alone, or with complexes of PPEUM with RK113 or the control mAb, PPEUM alone showed brighter staining than RK113/PPEUM complexes. Presumably, saturation of DC-SIGN receptors during pre-incubation with mAb RK113 inhibited binding of RK113/PPEUM complexes in the subsequent incubation step, confirming that targeting to DC surface receptors was critical for most of fusion protein binding.

### Internalization of fusion protein/mAb complexes

To find out whether anti-DC-SIGN mAb forming complexes with fusion proteins were internalized in the same way as anti-DC-SIGN mAb alone, we performed experiments in which DC were incubated with PPEUM or RK113 only, or with complexes of PPEUM with RK113 or with a second anti-DC-SIGN mAb (RK526) that is not internalized by DC.

Internalization of the fusion proteins was studied using the protocol described in Example 3 for studying internalization of anti-DC-SIGN mAbs.

After pre-incubation of cells with the fusion protein at 4°C, or with complexes of fusion proteins and mAb (5 µg/ml fusion protein and 10 µg/ml mAb, in PBS/glycerol, pre-incubated 1h at 4°C) cells were shifted to 37°C for various periods. At the end of 37°C incubations, cells were fixed with paraformaldehyde, and stained as above with PE-labeled goat antibodies to mouse Ig in order to detect mAb remaining on the cell surface, and analyzed for green and red fluorescence.

Figure 6b shows a quantitative evaluation of this experiment. The legend above the panels indicates: incubation at 4°C/incubation at 37°C.

As expected, complexes between RK113 and PPEUM were internalized, while those between RK526 and PPEUM were not. Therefore, binding of the fusion protein to the mAb did not alter the signals delivered through mAb binding to the DCSIGN receptor.

Internalization of RK113/PPEUM was also confirmed by fluorescent microscopy (not shown). While the fusion protein PPEUM alone remained surface associated after incubation for 1h at 37°C, RK113/PPEUM complexes were found in endolysosomal compartments identified by simultaneous staining with an antibody to the lysosome marker DC-LAMP.

### Example 6 : In vitro and in vivo presentation of targeted fusion proteins by MHC class I and class II molecules

To study delivery of fusion proteins to antigen processing compartments and resulting peptide presentation, we used OVA fusion proteins, taking advantage of OT-1 and OT2 transgenic T cells that recognize OVA peptides presented by MHC class I and class 11 molecules, respectively, of the H-2^{b} haplotype with high affinity.

### Antigen presentation assays in vitro

Bone marrow cells were prepared by flushing large bones from C57B1/ (H-2^{b}), 129 (H2^{b}), or Balb/c (H-2^{d}) mice. Cells filtered through a 40 µM strainer were then cultured in IMDM for 6 to 13 d with 10% FCS, supplemented with 20% supernatant from J558 transfectants secreting GM-CSF. OVA-specific T cells recognizing a peptide in the context of H-2K^{b} (OT-1) (HOGQUIST et al., Cell, 76, 17-27, 1994) or of 1-A^{b} (OT-2) (BARNDEN et al., Immunol Cell Biol, 76, 34-40, 1998), respectively, were prepared from lymph nodes of corresponding transgenic mice. T cells were either used directly, as naive cells, or, in order to produce "effector" OT-1 cells, activated in vitro by incubation with antigenic peptide-pulsed (10⁻⁶ M) irradiated spleen cells followed by culture (starting at 72h) in growth medium (complete RPMI supplemented with 10% supernatant from concanavalin A-stimulated rat spleens).

To study antigen presentation, DCs suspended in AIMV medium (Invitrogen) with 10% FCS were seeded at 50 µl and 100,000 cells per well in 96-well plates and incubated overnight with antigen. MAb/fusion protein complexes were formed by pre-incubating the two components, generally at a 1:1 molar ratio, for 1h at 4°C. The following antibodies were used: rat anti-mouse DEC-205 (clone NLDC-145), hamster anti-mouse CD11c (clone N418) and control mAb mouse anti HLA-A2 (clone BB7.2) were purchased from ATCCC (Manassas, VA) and purified from hybridoma supernatant; rat anti-mouse TLR2 and rat anti-mouse CD206 (mannose receptor 1) were from Serotec (Cergy Saint-Christophe, France); control mAb mouse anti-human TAP1 (clone 148.3) was purified from ascites. After o/n pulsing with complexes or with OVA (Worthington, Lakewood, NJ), DC were washed twice in medium before addition of 100,000 naive or effector T cells in 100 µl AIM-V to each well. Cytokine secretion by T cells was analyzed using commercial sandwich ELISA (BD Biosciences, Le Pont de Claix, France) as follows: OT-1 effectors: IFN-γ after 24h; naive OT-1: IL-2 after 24h or IFN-γ after 72h; naive OT-2: IL-2 after 48h.

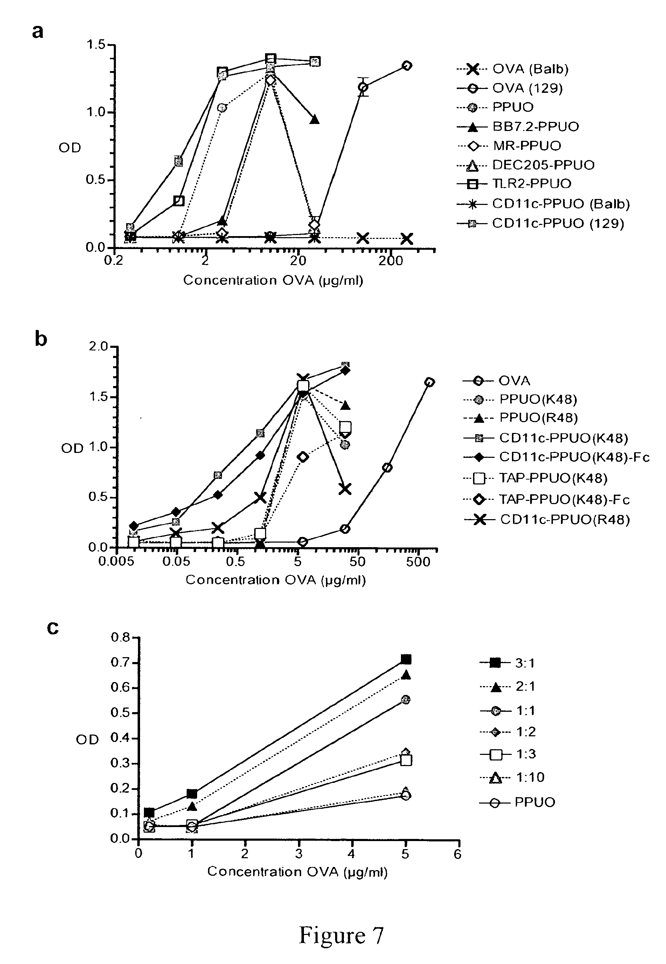

Figure 7a shows the results of an experiment where 1x10⁵ effector OT-1 T cells were stimulated for 24h with an equal number of bone marrow DC pre-pulsed overnight with indicated antigens, before measurement of IFN-γ in the supernatant by commercial sandwich ELISA. Where the mouse strain is not indicated in brackets, DCs were from a 129 mouse. Antigen complexes were formed with a molar ratio of 1:1 between mAb (specificity indicated before dash) and PPUO (K48).

Figure 7b shows the results of an equivalent experiment which was performed with 129 DC; however the ratio between mAb (anti-CD11c) and PPUO (K48) was varied as indicated (mAb:PPUO). In this experiment, naive OT-1 T cells were used, and IL-2 secretion was measured in supernatants removed after 24h addition of T cells to DC.

Figure 7c shows the results of an experiment in which naive lymph node OT-2 cells were stimulated with 129 DC pulsed with the shown antigens, and IL-2 secretion after 48 h was measured. Antigen specificity is indicated like in panel a. "Fc" indicates that Fc receptors were blocked by pre-incubation with mAb 2.4G2.

While OVA concentrations of 200 to 500 µg/ml were required for optimal stimulation of OT-1 and OT-2 cells by bone marrow DCs, substantially lower concentrations of the PPUO fusion proteins were sufficient for the same effect (Fig. 7a and c). Complexes between antibodies to CD11c or TLR2 (the latter for OT-1) and PPUO were most efficient (about 100-fold better than OVA), however several other antibodies including control antibodies, and PPUO alone, were also far superior to OVA in inducing MHC class I and class II presentation. Blocking of Fc receptors reduced only partly presentation of control mAb/PPUO complexes (Fig. 7c). Interestingly, even at higher antigen concentrations, a molar excess of targeting antibody resulted in optimal PPUO cross-presentation by MHC class I molecules, suggesting that fusion protein-mediated cross-linking of receptor-bound mAb may enhance presentation by MHC class I molecules (Fig. 7b).

### T cell priming in vivo

Naive OT-1 or OT-2 lymph node T cells were labeled at 2.5 x 10⁸ cells/ml with 10 µM carboxyfluorescein diacetate succinimidyl ester (CFSE; Invitrogen) for 12 min at 37°C, washed, and adjusted to a concentration of 1-2 x 10⁶/ml. C57B1/6 mice were anesthesized with 350 µl of 2.5% avertin and injected i.v. with 100 µl of T cells. Twenty-four h later, mice were injected under anesthesia in the four footpads with antigen. Three d later, single cell suspensions were prepared from draining lymph nodes and stained with a cocktail of anti-CD8-APC plus anti-Vβ5-PE for OT-1, or with a cocktail of anti-CD4-APC plus anti-Vβ5-PE for OT-2 (all antibodies from BD-Biosciences). For flow cytometric analysis, transgenic T cells were identified both by staining with these cocktails and in a forward scatter/side scatter dot plot. Proliferation of CFSE-labeled cells was quantified as number of mitoses per 5,000 gated T cells, using a FACSCalibur cytometer and FlowJo 6.4.7 (Treestar, San Carlos, CA) software.

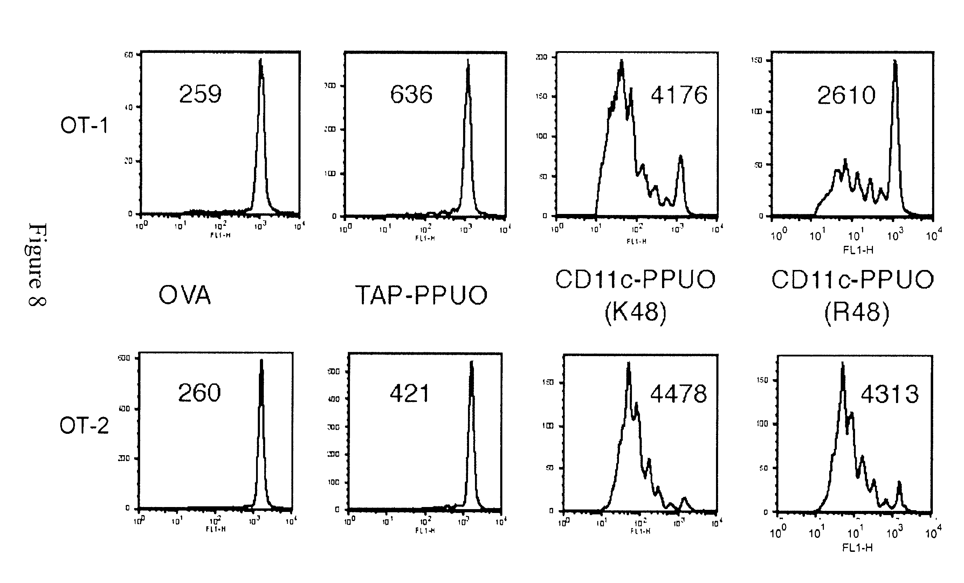

The results are illustrated by Figure 8 and Table 2.

The panels in Figure 8 show the proliferation of adoptively transferred, CFSE-labeled OT-1 (top) or OT-2 (bottom) T cells recovered three days after antigen injection from the lymph nodes of injected mice. Animals were injected with 200 ng of OVA or equivalent amounts of fusion proteins, as shown. The numbers in the panels indicate the number of mitotic events calculated per 5,000 CFSE-labeled T cells, using the FlowJo software. All panels are representative of at least two experiments.

Table 2 represents the proliferation of CFSE-labeled OT-1 T cells measured as number of mitotic events in lymph node T cells, recovered 72h after antigen injection. Numbers with an asterisk represent means derived from several experiments

**Table 2**

| Antigen | Amount (µg) | Antibody | Mitotic Events |
|---|---|---|---|
| - | - | - | 265* |
| Ovalbumin | 0.2 | - | 259 |
| Ovalbumin | 2.5 | - | 1270 |
| Ovalbumin | 5.0 | - | 2022 |
| Ovalbumin | 50 | - | 4498 |
| Ovalbumin | 250 | - | 4727 |
| PPUO (K48) | 0.05 | - | 423 |
| PPUO (K48) | 0.5 | - | 1523 |
| PPUO (K48) | 0.2 | TAP1 | 1087* |
| PPUO (K48) | 0.04 | CD11c | 1238 |
| PPUO (K48) | 0.05 | CD11c | 1983 |
| PPUO (K48) | 0.2 | CD11c | 3937* |
| PPUO (K48) | 0.5 | CD11c | 4514 |
| PPUO (K48) | 2.5 | CD11c | 4631 |
| PPUO (R48) | 0.04 | CD11c | 964 |
| PPUO (R48) | 0.2 | CD11c | 3058* |
| PPUO (K48) | 0.05 | DEC-205 | 562 |
| PPUO (K48) | 0.5 | DEC-205 | 2892 |
| PPUO (K48) | 0.05 | MR | 340 |
| PPUO (K48) | 0.5 | MR | 3246 |
| PPUO (K48) | 0.05 | TLR-2 | 538 |
| PPUO (K48) | 0.5 | TLR-2 | 3597 |

These results of *in vivo* priming of adoptively transferred naive T cells confirm the superior performance of the fusion proteins. Two hundred ng of PPUO/anti-CD11c complexes induced maximum proliferation of labeled class I and class 11 restricted T cells, while the same amount of OVA had no effect (Fig. 4d). Again, targeting to CD11c was most efficient, but targeting to several other DC receptors was also far superior to OVA in priming of OT-1 T cells (Table 2). Fusion protein alone, or in complex with a control mAb to an intracellular antigen were considerably less efficient though still more efficient than OVA. OVA fusion proteins carrying Lys and Arg residues at position 48 of the Ub performed similarly, although the former may be somewhat more efficient.

The demonstration that the OVA fusion proteins stimulate CD4⁺ and CD8⁺ T cell immunity suggests that the novel reagents may indeed be useful as vaccine components. Optimal stimulation of both cell types was achieved after injection of 0.2 to 0.5µg of antigen; a similar range (0.05 to 0.25µg) has previously reported for OVA coupled chemically to a monovalent mAb against DEC205 (BONIFAZ et al., J Exp Med, 199, 815-24, 2004).

These results indicate that fusion protein binding to mAb is compatible with efficient DC attachment, internalization and intracellular routing of mAb and coupled antigen.

Fusion proteins alone, or in complex with a control mAb, also outperformed OVA in stimulation of CD4⁺ and CD8⁺ cells, although this effect was much more pronounced *in vitro* than *in vivo,* were coupling to targeting mAb was far superior. While it is unclear what effect, if any, this phenomenon, presumably due to Fc receptor mediated internalization of fusion protein, has on the outcome of vaccination, using an excess of targeting mAb should reduce or even eliminate it.

### CONCLUSION

Although containing hydrophobic proteins, derived from several important pathogens, the novel fusion proteins described above are secreted by insect cells and can easily be purified by a single chromatographic step. They form complexes with mAb that allow their targeting to, and subsequent internalization by, DCs. Most importantly, a model fusion protein comprising ovalbumin elicits both CD4⁺ and CD8⁺ T cell responses *in vitro* and *in vivo* at least 100-fold more efficiently than ovalbumin alone, demonstrating the potential of the strategy for vaccination.

Thus, the data presented above indicate that the novel reagents may be highly efficient reagents for protein targeting to various cellular targets, an example of which is vaccine targeting to DCs.

In the latter context, we envisage that the reagents should be useful for complementing recombinant antibody/antigen complexes as vaccines (TRUMPFHELLER et al., J Exp Med, 203, 607-17, 2006), and help in evaluating rapidly various mAb and different receptors as targeting devices. Since, among the components of the fusion proteins, only the 50 amino acid protein G domains will be immunogenic in humans, sequential coupling of a fusion protein vaccine to different targeting mAb during repeat immunizations may also be helpful to prevent immunization against allo- and/or idiotypic determinants of targeting mAb.

## Claims

1. A fusion protein **characterised in that** it comprises:
- an IgG binding moiety consisting of two IgG binding domains of streptococcal protein G placed in tandem arrangement and optionally separated by a peptide linker;
- a cargo moiety comprising a polypeptide of interest.

2. A fusion protein of claim 1, wherein the cargo moiety comprises an ubiquitin domain fused to the N-terminal end of the polypeptide of interest.

3. A fusion protein of any of claims 1 or 2, wherein the cargo moiety comprises a labeling polypeptide.

4. A recombinant polynucleotide comprising a sequence encoding a fusion protein of any of claims 1 to 3.

5. A recombinant polynucleotide of claim 4, further comprising a sequence encoding a signal peptide.

6. A recombinant vector comprising a polynucleotide of any of claims 4 or 5.

7. An host cell comprising a polynucleotide of any of claims 4 or 5.

8. A method for producing a fusion protein of any of claims 1 to 3, **characterised in that** said method comprises culturing an host-cell of claim 7, and recovering said fusion protein produced by said host-cell.

9. A complex of a fusion protein of any of claims 1 to 3 with a mammalian IgG antibody.

10. A complex of claim 9, wherein said IgG antibody is directed against the dendritic cells receptor DC-SIGN.

11. A complex of claim 10, wherein said IgG antibody is the monoclonal antibody RK113D2 produced by the hybridoma CNCM 1-3661.

12. The monoclonal antibody RK113D2 produced by the hybridoma CNCM 1-3661.

13. A composition comprising a fusion protein of any of claims 1 to 3, a monoclonal antibody of claim 12, or a complex of any of claims 9 to 11.

14. A composition of claim 13, which is a pharmaceutical composition.

15. A pharmaceutical composition of claim 14, which is a vaccine.

16. A method for delivering a fusion protein of any of claims 1 to 3 to a target cell *in vitro,* wherein said method comprises forming a complex between a fusion protein of the invention and a mammalian IgG antibody directed against a surface receptor of said target cell, and contacting said complex with said target cell.

17. The method of claim 16, wherein said target cell is a dendritic cell.

18. The method of claim 17 which is used for preparing a dendritic cell vaccine.
